Europäisches Patentamt

European Patent Office

Office européen des brevets

(11) Veröffentlichungsnummer: **0 065 706**
B1

(19)

(12) # EUROPÄISCHE PATENTSCHRIFT

(45) Veröffentlichungstag der Patentschrift:
16.01.85

(21) Anmeldenummer: 82104155.5

(22) Anmeldetag: 12.05.82

(51) Int. Cl.⁴: **C 07 C 55/02,** C 07 C 51/34,
C 07 C 69/34, C 07 C 49/403,
C 07 C 45/66

(54) **Verfahren zur Herstellung von 3,3-Dimethylglutarsäure oder deren Ester.**

(30) Priorität: 27.05.81 CH 3474/81

(43) Veröffentlichungstag der Anmeldung:
01.12.82 Patentblatt 82/48

(45) Bekanntmachung des Hinweises auf die Patenterteilung:
16.01.85 Patentblatt 85/3

(84) Benannte Vertragsstaaten:
AT BE CH DE FR GB IT LI LU NL SE

(56) Entgegenhaltungen:
CHEMICAL ABSTRACTS, Band 86, Nr. 7, 14. Februar
1977, Seite 488, Nr. 43144t, Columbus Ohio (USA), M.
TICHY: "Simple synthesis of 3-t-butylglutaric acid"
CHEMICAL ABSTRACTS, Band 74, Nr. 5, 1. Februar
1971, Seite 280, Nr. 22472k, Columbus Ohio (USA), G.P.
POLLINI et al.: "5,5,6-Trimethyl-1,3-cyclohexaned
Ione(methydimedone) and 2,3,3-trimethylglutaric acid"

(73) Patentinhaber: LONZA AG, Gampel/Wallis (CH)

(72) Erfinder: Lehky, Pavel, Dr., Dammweg 13, Naters
(Kanton Wallis) (CH)

(74) Vertreter: Weinhold, Peter, Dr. et al, Patentanwälte Dr. V.
Schmied-Kowarzik Dipl.-Ing. G. Dannenberg Dr. P.
Weinhold Dr. D. Gudel Dipl.-Ing. S. Schubert Dr. P. Barz
Siegfriedstrasse 8, D-8000 München 40 (DE)

# Beschreibung

Die Erfindung betrifft ein Verfahren zur Herstellung von 3,3-Dimethylglutarsäure oder deren Ester aus Dimedon.

3,3-Dimethylglutarsäure und ihre Ester stellen Zwischenprodukte bei der Herstellung von Pestiziden (DE-OS Nr. 2813341) dar. Ferner sind sie als Zusatz für Schmieröl geeignet (US-PS Nr. 2971915).

Für die Herstellung von 3,3-Dimethylglutarsäure sind aus der Literatur verschiedene Methoden bekannt. So wird nach G. Komppa, „Ann.", *368*, 126 (1909), W.T. Smith und G.L. McLeod, „Org. Synthesis", *31*, 40 (1951) und J. Walker und J.K. Wood, „J. Chem. Soc.", *89*, 598 (1906) durch Oxidation von Dimedon (5,5-Dimethyl-1,3-cyclohexandion) mit Natriumhypochlorit 3,3-Dimethylglutarsäure erhalten. L.P. Vinogradova *et al.*, „Chem. Abstr.", *56*, 338 (1962) und K. Ruhl, „Z. für Naturf.", *4B*, 199 (1949) beschreiben die Oxidation von Dimedon mit Wasserstoffperoxid. Beiden Verfahren haften Nachteile an. Das erstere benötigt eine grosse Menge Hypochloritlösung und produziert u.a. viel anorganischen Abfall, und beim letzteren ist der grosse Verbrauch an teurem Wasserstoffperoxid ein ernst zu nehmendes Hemmnis.

Ziel der Erfindung ist es, 3,3-Dimethylglutarsäure, ausgehend von einem billigen Edukt, auf einfache Weise in hoher Reinheit zu produzieren, so dass keine aufwendigen Reinigungsverfahren nachgeschaltet werden müssen.

Erfindungsgemäss wird dies dadurch erreicht, dass man Dimedon mit Ozon in Gegenwart eines unter den Reaktionsbedingungen in Gegenwart eines unter den Reaktionsbedingungen inerten Lösungsmittels in ein Ozonanlagerungsprodukt überführt und dieses durch Hydrolyse in die 3,3-Dimethylglutarsäure oder durch Alkoholyse zu deren Ester umsetzt.

Wird als synthetisierendes Produkt 3,3-Dimethylglutarsäure angestrebt, so führt man die Hydrolyse des Ozonanlagerungsproduktes vorteilhaft mit Hilfe von heissem, vorzugsweise mit kochendem Wasser durch.

Ist das Endprodukt ein 3,3-Dimethylglutarsäureester, so wird die Spaltung des Ozonanlagerungsproduktes unter gleichzeitiger Esterbildung mit Alkoholen in Gegenwart einer Mineralsäure durchgeführt. Der verwendete Alkohol muss dabei mit dem Alkoholteil des gewünschten Esters übereinstimmen.

Die Ozonolyse wird vorteilhaft bei Temperaturen von −80 bis +40, vorzugsweise bei −10 bis +20°C, durchgeführt. Die Reaktionszeit hängt zur Hauptsache von der Leistung des Ozongenerators ab. Mittlere Reaktionszeiten liegen etwa bei 0,5 bis 5 h.

Als Ausgangsmaterial kann auch ein Dimedon eingesetzt werden, welches durch Zyklisierung aus 3,3-Dimethyl-5-oxohexansäureester mit Natriumalkoholat hergestellt wird und somit nicht isoliert in einer alkoholischen Lösung vorliegt.

Wird von isoliertem Dimedon ausgegangen und ist ein 3,3-Dimethylglutarsäurediester das gewünschte Produkt, so können anstelle von einem Alkohol auch andere Lösungsmittel eingesetzt werden, mit der Einschränkung, dass diese Lösungsmittel nicht mit Alkoholen oder Estern unter den Bedingungen der Reaktion reagieren dürfen. Geeignete Lösungsmittel sind Ester (z.B. Äthylacetat), Chlorkohlenwasserstoffe, wie Methylenchlorid, oder Kohlenwasserstoffe. Bei Verwendung von solchen Lösungsmitteln muss dann nach Bildung des Dimedonozonanlagerungsproduktes der geeignete Alkohol zugesetzt werden.

Wird als Reaktionsprodukt 3,3-Dimethylglutarsäure gewünscht, so wird nach einer speziellen Ausführungsform der Erfindung nach Beendigung der Ozonolyse und der nachfolgenden Hydrolyse das Reaktionsgemisch auf Raumtemperatur abgekühlt und das Produkt mit Äther extrahiert und letzterer anschliessend abgedampft. Nach der Trocknung erhält man weisse kristalline, rohe Dimethylglutarsäure mit einer Reinheit von 85%. Durch Waschen mit wenig Toluol und Hexan wird die Reinheit auf 98% erhöht.

Aus dem Filtrat lassen sich noch geringe Mengen des 3,3-Dimethylglutarsäuremonomethylesters isolieren, welche wieder in den Prozess eingeschleust werden können. Berücksichtigt man die Wiederverwendbarkeit dieses Nebenproduktes, so beträgt die Selektivität 90 bis 91%.

Wird als Reaktionsprodukt ein Ester der 3,3-Dimethylglutarsäure gewünscht, so wird das Ozonanlagerungsprodukt mit einem Alkohol, vorzugsweise in Gegenwart katalytischer Mengen einer Mineralsäure, umgesetzt. Die Zugabe des Alkohols erfolgt spätestens nach der Bildung des Ozonanlagerungsproduktes.

Nach beendeter Alkoholyse kann die Reaktionslösung auf Raumtemperatur gekühlt, mit einer Base, z.B. Natronlauge, gelöst in einem Alkohol, neutralisiert und durch Destillation aufgetrennt werden.

Man erhält die Diester der 3,3-Dimethylglutarsäure mit einer Reinheit von über 97%.

*Beispiel 1:*

Man löste 4,8 g metallisches Natrium in 100 ml wasserfreiem Methanol und erwärmte zum Rückfluss. Hierzu wurden 30,0 g 3,3-Dimethyl-5-oxohexansäuremethylester, welcher aus der Umsetzung von Isophoron mit Ozon stammte und einen Gehalt von 98,1% aufwies, im Verlauf von 0,5 h zugetropft. Anschliessend wurden weitere 2 h rückflussiert. Nach dem Abkühlen säuerte man mit konz. Schwefelsäure auf pH 2 an. Das ausgefallene Natriumsulfat wurde abfiltriert; diese Filtration konnte durch die Zugabe von 2,0 g eines Filterhilfsmittels (z.B. Clarcel® Typ Dic 3) erleichtert werden. Der Filterrückstand wurde mit 100 g Methanol nachgewaschen. Die anfallende Lösung enthielt laut analytischer Bestimmung 22,64 g Dimedon. Diese Lösung von Dimedon in Methanol wurde auf −2 bis 0°C abgekühlt. Anschliessend wurde ein Strom von ca. 4% Ozon in Sauerstoff zwecks Herstellung eines Ozonanlagerungsproduktes in diese Lösung bis zu deren Sättigung eingeleitet.

Nach beendeter Ozonolyse wurde die Lösung des entstandenen Ozonanlagerungsproduktes in 200 ml siedendes Wasser eingetropft und während 3 h auf Rückflusstemperatur erhitzt. Nach der Hydrolyse wurde das Ganze auf Raumtemperatur abgekühlt, dreimal mit 80 ml Äther extrahiert, der Äther aus dem Extrakt durch Verdampfen abgerieben und die sich gebildeten Kristalle der rohen 3,3-Dimethylglutarsäure getrocknet. Die Reinheit betrug 85% (nach GC).

Anschliessend wurde diese rohe 3,3-Dimethylglutarsäure mit 20 ml Toluol und 20 ml Hexan auf einer Nutsche nochmals gewaschen. Dadurch konnte die Reinheit des Produktes auf 98% erhöht werden. Als Resultat erhielt man nach der Trocknung 21,83 g weisse Kristalle von 3,3-Dimethylglutarsäure 98,0%ig, Smp. 100,5 bis 101,5°C. Die Ausbeute, auf das Dimedon berechnet, betrug 82,7% und auf eingesetzten 3,3-Dimethyl-5-oxohexansäuremethylester 78,1%.

Als Nebenprodukt wurden 2,28 g 3,3-Dimethylglutarsäuremonomethylester erhalten, welche wieder in die Synthese eingesetzt werden konnten. Berücksichtigt man diesen Umstand, so betrug die Selektivität der Reaktion 85,8%, bezogen auf umgesetzten 3,3-Dimethyl-5-oxohexansäuremethylester.

*Beispiel 2:*

Eine Lösung, hergestellt aus 14,0 g festem Dimedon und 110 g Methanol, wurde wie in Beispiel 1 ozonolysiert. Nach beendeter Ozonolyse wurde der Lösung des entstandenen Ozonanlagerungsproduktes 0,92 g konz. Schwefelsäure langsam zugegeben. Die saure Lösung wurde 3 h lang unter Rückfluss gekocht. Nach der Alkoholyse wurde die Lösung auf Raumtemperatur abgekühlt und mit Natriumhydroxid neutralisiert. Das Methanol wurde abdestilliert und anschliessend der 3,3-Dimethylglutarsäuredimethylester durch Vakuumdestillation (90°C/14 mbar) isoliert.

Man erhielt 16,8 g farblose Flüssigkeit, die laut Gaschromatographie 98,0% des 3,3-Dimethylglutarsäuredimethylesters enthielt. Dies entsprach einer Ausbeute von 87,6%, auf das eingesetzte Dimedon berechnet.

*Beispiel 3:*

Eine Lösung, hergestellt aus 14,0 g festem Dimedon, 55,0 g Eisessig und 55,0 g Äthylacetat, wurde wie in Beispiel 1 ozonolysiert und durch Eintropfen in 200,0 g siedendes Wasser hydrolysiert. Das Hydrolysat wurde bis zur Trockene eingedampft. Die gebildeten Kristalle der rohen 3,3-Dimethylglutarsäure wurden mit 20 ml Toluol und 20 ml Hexan auf einer Nutsche gewaschen. Dadurch konnte die Reinheit des Produktes auf 98,1% erhöht werden. Nach der Trocknung erhielt man 13,9 g weisse Kristalle von 3,3-Dimethylglutarsäure (Smp. 101 bis 102°C), was 13,6 g 3,3-Dimethylglutarsäure 100%ig entsprach. Die Ausbeute, auf eingesetztes Dimedon berechnet, betrug 85,0%.

## Patentansprüche

1. Verfahren zur Herstellung von 3,3-Dimethylglutarsäure oder deren Ester aus Dimedon, dadurch gekennzeichnet, dass man Dimedon in Gegenwart eines unter den Reaktionsbedingungen inerten Lösungsmittels mit Ozon in ein Ozonanlagerungsprodukt überführt und dieses durch Hydrolyse in die 3,3-Dimethylglutarsäure oder durch eine Alkoholyse in deren Ester umsetzt.

2. Verfahren zur Herstellung der 3,3-Dimethylglutarsäure gemäss Patentanspruch 1, dadurch gekennzeichnet, dass man die Hydrolyse in heissem Wasser, vorzugsweise in siedendem Wasser, durchführt.

3. Verfahren zur Herstellung der 3,3-Dimethylglutarsäureester gemäss Patentanspruch 1, dadurch gekennzeichnet, dass man das Ozonanlagerungsprodukt durch Behandlung mit einem Alkohol in Gegenwart einer Mineralsäure in einen 3,3-Dimethylglutarsäureester überführt.

4. Verfahren gemäss Patentanspruch 1, dadurch gekennzeichnet, dass man die Ozonolyse bei Temperaturen von −80 bis +40, vorzugsweise von −10 bis +20°C, durchführt.

5. Verfahren gemäss den Patentansprüchen 1 bis 4, dadurch gekennzeichnet, dass man als Ausgangsprodukt ein Dimedon verwendet, welches durch Zyklisierung aus 3,3-Dimethyl-5-oxohexansäureester mit Natriumalkoholat hergestellt wird.

## Claims

1. Process for the preparation of 3,3-dimethylglutaric acid or its esters from dimedone, characterized in that dimedone in the presence of a solvent inert under the reaction conditions is converted with ozone into an ozone addition product, and reacting same by hydrolysis into 3,3-dimethylglutaric acid, or by alcoholysis into its esters.

2. Process for the preparation of 3,3-dimethylglutaric acid according to Claim 1, characterized in that the hydrolysis is carried out in hot water, preferably in boiling water.

3. Process for the preparation of 3,3-dimethylglutaric acid according to Claim 1, characterized in that the ozone addition product is converted by treating it with an alcohol in the presence of a mineral acid into a 3,3-dimethylglutaric acid ester.

4. Process according to Claim 1, characterized in that the ozonolysis is carried out at temperatures of from −80 to +40°C, preferably of from −10 to +20°C.

5. Process according to Claims 1 to 4, characterized in using as starting product a dimedone which has been obtained by cyclization from 3,3-dimethyl-5-oxohexanoic ester with sodium alkoxide.

## Revendications

1. Procédé pour la préparation de l'acide 3,3-diméthylglutarique ou de ses esters à partir du dimédon, caractérisé en ce qu'on transforme le dimédon en présence d'un solvant inerte dans les conditions de la réaction au moyen d'ozone en un produit de fixation de l'ozone et on transforme celui-ci par hydrolyse en l'acide 3,3-diméthylglutarique ou par une alcoolyse en ses esters.

2. Procédé pour la préparation de l'acide 3,3-diméthylglutarique selon la revendication 1, caractérisé en ce qu'on effectue l'hydrolyse dans de l'eau chaude, de préférence dans de l'eau bouillante.

3. Procédé pour la préparation des esters de l'acide 3,3-diméthylglutarique selon la revendication 1, caractérisé en ce qu'on transforme le produit de fixation de l'ozone en un ester de l'acide 3,3-diméthylglutarique par traitement au moyen d'un alcool en présence d'un acide minéral.

4. Procédé selon la revendication 1, caractérisé en ce qu'on effectue l'ozonolyse à des températures de −80 à +40°C, de préférence de −10 à +20°C.

5. Procédé selon les revendications 1 à 4, caractérisé en ce que l'on utilise, en tant que produit de départ, un dimédon qui est préparé par cyclisation d'un ester de l'acide 3,3-diméthyl-5-oxohexanoïque au moyen d'un alcoolate de sodium.